# EUROPÄISCHE PATENTSCHRIFT

(11) **EP 2 247 932 B1**
(45) Veröffentlichungstag und Bekanntmachung des Hinweises auf die Patenterteilung: **14.12.2011**
(21) Anmeldenummer: 09714850.6
(22) Anmeldetag: 25.02.2009
(51) Int. Cl.: G01M 3/20, H01J 41/06, G01N 27/62, G01N 33/00

(54) **HELIUMSENSOR**
HELIUM SENSOR
DÉTECTEUR D'HÉLIUM

(30) Priorität: 28.02.2008 DE 102008011686
(43) Veröffentlichungstag der Anmeldung: 10.11.2010
(73) Patentinhaber: Inficon GmbH, 50968 Köln (DE)
(72) Erfinder: GROSSE BLEY, Werner, 53125 Bonn (DE); WETZIG, Daniel, 50999 Köln (DE)
(74) Vertreter: von Kreisler Selting Werner
(86) Internationale Anmeldenummer: PCT/EP2009/052225
(87) Internationale Veröffentlichungsnummer: WO 2009/106543

(56) Entgegenhaltungen:
- DE-A1- 10 031 882
- GB-A- 1 031 766
- US-A- 3 684 401

## Beschreibung

Die Erfindung betrifft einen Heliumsensor mit einer Detektionskammer, die eine für ein Testgas selektiv durchlässige Wand aufweist, einer Ionengetterpumpe mit mindestens einer Katode, mindestens einer Anode, die an eine Spannungsquelle angeschlossen sind, und einem Magnetfelderzeuger, der ein die Detektionskammer durchquerendes Magnetfeld erzeugt.

Ein derartiger Heliumsensor ist in DE 100 31 882 A1 (Leybold Vacuum GmbH) beschrieben. Die Detektionskammer besteht aus Glas und die für das Testgas Helium selektiv durchlässige Wand ist eine Siliziumscheibe, die mit Durchbrechungen versehen ist, welche jeweils mit einer dünnen Quarzmembran verschlossen sind und eine spezielle Heizung aufweist. Ein derartiger Gassensor wird wegen der Beschaffenheit der selektiv durchlässigen Wand auch als Quarzfenstersensor bezeichnet. Die Gasatome, die die selektiv durchlässige Wand passiert haben, werden in der Detektionskammer ionisiert und durch ein elektrisches Feld zu einer Katode geführt und dort gebunden.

Ein ähnlicher Gassensor ist beschrieben in DE 10 2004 034 381 A1 (Inficon GmbH).

Bei einem Gassensor, der hier in Rede stehenden Art erfolgt die Detektion des Testgases dadurch, dass beim Entladen von ionisierten Gasatomen und Absorbieren in der Katode in dem elektrischen Stromkreis ein Strom erzeugt wird, der detektierbar ist. Die Nachweisgrenze eines Heliumsensors auf Basis der Quarzfenstertechnik ist dadurch beschränkt, dass ein Basisstrom mit Instabilitäten in Form von Drift und Rauschen existiert. Rauschen entsteht durch den Adsorptions-Desorptions-Vorgang an der Katode des Kaltkatodensystems, weil nicht sichergestellt werden kann, dass einmal absorbierte Teilchen dauerhaft festgehalten werden. Je nach Katodentemperatur und Einwirkung von auftreffenden Atomen verlassen auch wieder Teilchen die Katode und erzeugen eine gewisse Instabilität des Basisstroms, und somit Rauschen. Eine Drift entsteht im wesentlichen temperaturabhängig dadurch, dass sich wegen der ungenügenden Bindungskräfte bei veränderter Temperatur ein neues Gleichgewicht der Bedeckung bzw. Lösung im Katodenmaterial einstellt.

Der Erfindung liegt die Aufgabe zugrunde, einen Heliumsensor der eingangs genannten Art mit verbesserter Nachweisgrenze zu schaffen.

Der Heliumsensor der vorliegenden Erfindung ist durch den Patentanspruch 1 definiert. Er zeichnet sich dadurch aus, dass mindestens eine Katode des Kaltkatodensystems Beryllium enthält.

Im Stand der Technik bestehen die Katoden des Kaltkatodensystems aus Titan oder Tantal. Titan hat ein Atomgewicht von 48 amu und Tantal ein Atomgewicht von 181 amu. Das Atomgewicht von Helium beträgt 4 amu. "amu" bedeutet, die Rumpfatommasse (atomar mass unit). Die Erfindung geht von dem Gedanken aus, dass bei einem hohen Atomgewicht des Katodenmaterials die leichten Heliumatome, die auf die Katode auftreffen, mit hoher Energie als Neutralteilchen reflektiert werden. Eine bessere Einbindung der Heliumatome in die Katode kann erfolgen, wenn man als Katodenmaterial ein Metall mit einem Atomgewicht ähnlich demjenigen von Helium verwendet. Das leichteste Metall, das sich noch verhältnismäßig gut industriell handhaben lässt, ist Beryllium mit einem Atomgewicht von 9 amu. Die Erfindung schlägt vor, mindestens eine Katode des Kaltkatodensystems ganz oder teilweise aus Beryllium zu fertigen.

Durch die Erfindung wird erreicht, dass nicht nur die Rauschinstabilität des elektrischen Signals reduziert wird, sondern der Basisstrom, der den Heliumrestdruck angibt, um ein bis zwei Größenordnungen niedriger ausfällt, da offenbar die gepumpten Heliumatome deutlich besser dauerhaft gebunden werden. Durch den niedrigen Basisstrom wird auch die thermische Drift um denselben Betrag reduziert, da sie prozentual vom Basisstrom abhängt.

Die Nachweisgrenze eines solchen Sensors, die definiert ist als Summe von Drift (pro Minute) und Rauschen (Spitze-Spitze), wird also entsprechend verbessert.

Bei dem erfindungsgemäßen Heliumsensor kann das Kaltkatodensystem entweder in Penning-Geometrie ausgeführt werden oder in MagnetronGeometrie. Beide Geometrien sind in DE 100 31 882 A1 beschrieben, deren Inhalt hiermit durch Verweisung in die vorliegende Beschreibung einbezogen wird.

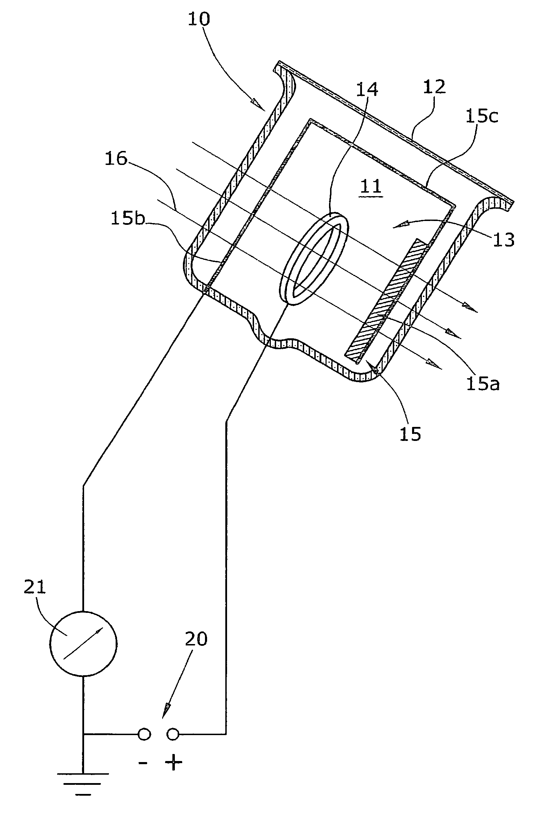

Im Folgenden wird unter Bezugnahme auf die einzige Figur der Zeichnung ein Ausführungsbeispiel der Erfindung näher erläutert.

Die Figur zeigt schematisch den Aufbau eines Quarzfenster-Heliumsensors.

Der Heliumsensor weist ein aus Glas bestehendes Gehäuse 10 auf, das eine evakuierte Detektionskammer 11 umschließt. Das Gehäuse 10 bildet einen quaderförmigen oder zylinderförmigen Körper mit geschlossener Bodenwand. Die der Bodenwand gegenüberliegende Öffnung ist mit einer selektiv durchlässigen Wand 12 verschlossen. Die Wand 12 besteht aus einer Quarzfenstermembran, wie sie in DE 100 31 882 A1 beschrieben ist. Während die Wand des Gehäuses 10 für Gas undurchlässig ist, ist die Wand 12 selektiv für das Testgas, hier Helium, durchlässig.

Die Detektionskammer 11 enthält eine Ionengetterpumpe 13. Diese weist in einer Penninggeometrie eine ringförmige Anode 14 und eine Katode 15 mit zwei parallelen Katodenschenkeln 15a, 15b auf. Die Anode 14 ist ringförmig ausgebildet, wobei die Ringebene parallel zu den Ebenen der Katodenschenkel 15a, 15b verläuft, zwischen denen die Anode angeordnet ist. Orthogonal zu dieser Ebene verläuft ein Magnetfeld 16, das durch einen außerhalb des Gehäuses 10 angeordneten (nicht dargestellten) Magnetfelderzeuger erzeugt wird. Hierbei handelt es sich beispielsweise um einen Permanentmagneten. Alternativ kann die Anordnung auch in Magnetrongeometrie ausgeführt werden, wobei die Katode (beim invertierten Magnetron die Anode) stabförmig in der Mittelachse eines Rohres angeordnet ist. Das Rohr bildet beim Magnetron die Anode (beim invertierten Magnetron die Katode), das Magnetfeld verläuft axial im Rohr und wird durch einen Ringmagneten außerhalb des Rohres in bekannter Weise erzeugt.

Zwischen Anode 14 und Katode 15 wird eine Hochspannung von etwa 3000 V gelegt, die von einer Spannungsquelle 20 erzeugt wird. Die Spannungsquelle 20 ist außerhalb des Gehäuses 10 angeordnet und über Stromdurchführungen, die durch die Gehäusewand hindurchgehen, mit der Katode und der Anode verbunden. Der Stromkreis enthält einen Strommesser 21, der den Katodenstrom misst und dessen Messwert ein quantitatives Maß für die die Wand 12 passierende Menge an Helium bildet.

Die in der Zeichnung dargestellte Geometrie der Ionengetterpumpe ist eine Penning-Geometrie.

Erfindungsgemäß besteht der eine Schenkel 15a der Katode 15 aus Beryllium. Der gegenüberliegende Schenkel 15b der Katode besteht aus Titan oder einem anderen schweren Metall, z. B. Tantal, wie auch das die beiden Schenkel 15a und 15b verbindende Katodenblech 15c. Auch eine Ausführung mit beiden Katodenschenkeln bzw. Magne-trongeometrie mit der Katode aus Beryllium hat denselben Effekt, wie Messungen gezeigt haben.

Beim Eintritt von Heliumatomen in die Detektionskammer 11 werden die Heliumatome ionisiert und durch das elektrische Feld in Richtung auf die Katode 15 beschleunigt. Dabei werden die Heliumionen in das leichtgewichtige Gefüge der Berylliumkatode eingebettet und gebunden. Wenn einer der beiden Katodenschenkel aus einem "schweren" Metall ist, werden die neutralisierten Ionen als Neutralteilchen reflektiert und können damit leicht in die gegenüberliegende Katode aus "leichtem" Metall eindringen und absorbiert werden. Als Folge hiervon ist der Anteil der reflektierten bzw. ungebundenen Heliumionen verringert. Dadurch wird der Basisstrom (durch die Neutralisierung von "gepumpten" Ionen) verringert und das Rauschsignal verbessert. Durch den verringerten Basisstrom ist auch die Drift dieses Stroms entsprechend geringer. Die Folge ist eine Verbesserung der Nachweisgrenze, d. h. des kleinsten nachweisbaren Heliumpartialdrucks (der sich aus der Summe von Drift und Rauschen ergibt).

## Patentansprüche

1. Heliumsensor mit einer Detektionskammer (11), die eine für Helium selektiv durchlässige Wand (12) aufweist, einer Ionengetterpumpe (13) mit mindestens einer Katode (15), mindestens einer Anode (14), die an eine Spannungsquelle (20) angeschlossen sind, und einem Magnetfelderzeuger, der ein die Detektionskammer durchquerendes Magnetfeld (16) erzeugt,
**dadurch gekennzeichnet,**
**dass** mindestens eine Katode (15) Beryllium enthält.

2. Heliumsensor nach Anspruch 1, **dadurch gekennzeichnet, dass** bei einer Ionengetterpume (13) mit zwei Katodenschenkeln (15a, 15b) zwischen denen die Anode (14) angeordnet ist, der eine Katodenschenkel (15a) Beryllium enthält und der andere ein schweres Metall wie Ta oder Ti.

3. Heliumsensor nach Anspruch 1 oder 2, **dadurch gekennzeichnet, dass** die mindestens eine Katode auf ihrer der Anode zugewandten Seite vollständig aus Beryllium besteht.

4. Heliumsensor nach einem der Ansprüche 1 - 3, **dadurch gekennzeichnet, dass** die Ionengetterpumpe in Penning-Geometrie ausgeführt ist.

5. Heliumsensor nach einem der Ansprüche 1 - 3, **dadurch gekennzeichnet, dass** die Ionengetterpumpe in Magnetron- oder invertierter MagnetronGeometrie ausgeführt ist.

## Claims

1. A helium sensor comprising a detection chamber (11) with a wall (12) selectively permeable to helium, an ion getter pump (13) with at least one cathode (15) and at least one anode (14), which are connected to a voltage source (20), and a magnetic field generator generating a magnetic field (16) across the detection chamber,
**characterized in that**
at least one cathode (15) contains beryllium.

2. The helium sensor of claim 1, **characterized in that**, with an ion getter pump (13) having two cathode legs (15a, 15b) between which the anode (14) is arranged, one cathode leg (15a) contains beryllium and the other contains a heavy metal such as Ta or Ti.

3. The helium sensor of claim 1 or 2, **characterized in that** said at least one cathode is entirely made of beryllium on the side facing the anode.

4. The helium sensor of one of claims 1 - 3, **characterized in that** the ion getter pump is designed in a Penning geometry.

5. The helium sensor of one of claims 1 - 3, **characterized in that** the ion getter pump is designed in a magnetron geometry or in an inverted magnetron geometry.

## Revendications

1. Détecteur d'hélium comprenant une chambre de détection (11) avec une paroi (12) sélectivement perméable à hélium, une pompe ionique à sorbeur (13) avec au moins une cathode (15), au moins une anode (14), qui sont connectées à une source de voltage (20), et un générateur de champ magnétique générant un champs magnétique (16) à travers ladite chambre de détection,
**caractérisé en ce qu'**
au moins une cathode (15) contient de béryllium.

2. Détecteur d'hélium selon la revendication 1, **caractérisé en ce qu'**avec une pompe ionique à sorbeur (13) comprenant deux branches de cathode (15a, 15b) entre lesquelles est disposée ladite anode (14), l'une des branches de cathode (15a) contient de béryllium et l'autre contient un métal lourd comme Ta ou Ti.

3. Détecteur d'hélium selon les revendications 1 ou 2, **caractérisé en ce que** la côté de ladite au moins une cathode, tournée vers ladite anode, est complètement en béryllium.

4. Détecteur d'hélium selon l'une des revendications 1 - 3, **caractérisé en ce que** la pompe ionique à sorbeur est configurée selon la géométrie de Penning.

5. Détecteur d'hélium selon l'une des revendications 1 - 3, **caractérisé en ce que** la pompe ionique à sorbeur est configurée selon la géométrie d'un magnétron ou la géométrie inverse d'un magnétron.
